Europäisches Patentamt

European Patent Office

Office européen des brevets

(11) Publication number: **0 477 958 B1**

# EUROPEAN PATENT SPECIFICATION

(45) Date of publication of patent specification: **15.03.95**  (51) Int. Cl.6: **A61L 17/00**

(21) Application number: **91116480.4**

(22) Date of filing: **26.09.91**

The file contains technical information submitted
after the application was filed and not included in
this specification

(54) **Surgical sutures made from polyvinyl alcohol fibers.**

(30) Priority: **26.09.90 JP 257998/90**

(43) Date of publication of application:
**01.04.92 Bulletin  92/14**

(45) Publication of the grant of the patent:
**15.03.95 Bulletin  95/11**

(84) Designated Contracting States:
**DE FR GB NL**

(56) References cited:
**CH-A- 474 269**

(73) Proprietor: **UNITIKA LTD.**
**No. 50, Higashihonmachi 1-chome**
**Amagasaki-shi**
**Hyogo (JP)**

(72) Inventor: **Ikada, Yoshito**
**2-182, Gokashohirookadani**
**Uji-shi,**
**Kyoto (JP)**
Inventor: **Tomita, Naohide**
**814, Kiyamachi**
**Nabari-shi,**
**Mie (JP)**
Inventor: **Takagi, Kunihiko, c/o Unitika Ltd.**

**Central Research Institute,**
**23, Uji Kozakura**
**Uji-shi,**
**Kyoto (JP)**
Inventor: **Mochizuki, Masatsugu, c/o Unitika**
**Ltd.**
**Central Research Institute,**
**23, Uji Kozakura**
**Uji-shi,**
**Kyoto (JP)**
Inventor: **Nagata, Naohiko, c/o Unitika Ltd.**
**Central Research Institute,**
**23, Uji Kozakura**
**Uji-shi,**
**Kyoto (JP)**

(74) Representative: **Hansen, Bernd, Dr.**
**Dipl.-Chem. et al**
**Hoffmann, Eitle & Partner**
**Patent- und Rechtsanwälte,**
**Postfach 81 04 20**
**D-81904 München (DE)**

## Description

### FIELD OF THE INVENTION

This invention relates to surgical sutures made from polyvinyl alcohol (hereunder abbreviated as "PVA") fibers.

### BACKGROUND OF THE INVENTION

While surgical sutures are required to possess different characteristics depending on the site of use, high tensile strength and knot strength are particularly required when they are used in surgical operations on large abdominal walls, large blood vessels, heart, tendon, ligamentum, bone marrow and fascia, as well as in the transplanting of artificial organs. For fixing bones, even higher tensile strength and creep resistance are required, so metal wires are commonly used for this purpose. However, because of their excessive hardness, metals are difficult to handle and may occasionally destroy tissues when they are tied off or the surgeon may inadvertently injure his hand. As a further problem, since metal wires do not transmit X-rays and leave a shadow on X-ray film, they also impart discomfort to the patient. Under these circumstances, ligation with supple synthetic fibers is strongly desired.

Among the surgical sutures commonly used today, polyester and polypropylene fibers are two materials that can retain high strength in vivo over a prolonged period. However, both have a tensile strength of only about 5 g/d while their elongation at break is as high as about 20%, so they are not suitable for use in fixing bones, etc.

Increasing attention has been drawn to high strength polyethylene fibers and aramid fibers as materials having higher tensile strengths. However, because of the low friction between fibers, high strength polyethylene fibers have low knot retention and, with a stress of only about 1 g/d being applied to a knot, such fibers will slip from each other to cause the knot to untie. To retain the knot, at least seven throws of ligation or fixation with adhesives is necessary. Aramid fibers have low biocompatibility, a fatal defect for using them as suture materials.

PVA fibers have the highest strength and initial modulus of general purpose fibers. Further, they have low elongation at break and creep value and have high biocompatibility. Therefore, it would be quite beneficial if surgical sutures could be produced from PVA fibers.

The Swiss patent CH-A-474269 discloses fibres manufactured by preparing a PVA film, from an aqueous solution of PVA, having a molecular weight of 200000 or more, cutting narrow bands in the film and subjecting the bands to stretching, the fibres thus produced being used as surgical sutures. However, the strength of the fibres is relatively low at 50 kg/cm$^2$ (0.04 g/d) making satisfactory ligation difficult to achieve.

As described above, several synthetic fibers have been proposed for use as surgical sutures in sites where exceptionally high tensile strength, knot strength and knot retention are required, but such have their own disadvantages and have not yet been commercialized.

### SUMMARY OF THE INVENTION

The present invention has been achieved under these circumstances and has as an object providing a surgical suture made of PVA fibers that exhibits high tensile strength, knot strength and knot retention in vivo, which are inexpensive and which have good biocompatibility.

This object of the present invention is attained by a PVA surgical suture that comprises a braid of PVA fibers having a tensile strength of 12 to 25 g/d, a tensile elongation at break of 3.5 to 6% and a knot strength of 4 to 8 g/d, the suture having a knot retention of 3 to 8 g/d.

### BRIEF DESCRIPTION OF THE DRAWINGS

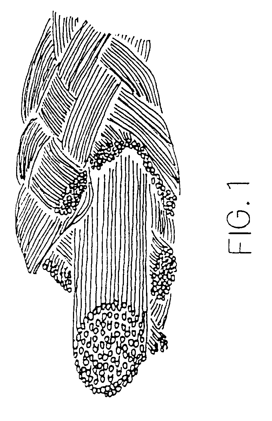

Figure 1 shows a magnified side view of braided suture comprising sheath and core.

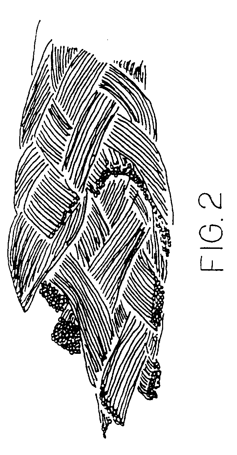

Figure 2 shows a magnified side view of braided suture without core.

### DETAILED DESCRIPTION OF THE INVENTION

The PVA fibers used as the material for making the surgical suture of the present invention are required to have a tensile strength of at least 12 g/d, preferably at least 14 g/d. If their tensile strength is lower than 12 g/d, the suture will readily break when it is used to tie off a tendon, ligamentum, periosteum and at other sites where high tension is exerted, and the objects of the present invention cannot be attained. The higher the tensile strength, the better, since not only is the reliability of suture improved but it also becomes possible to make fine sutures. In practice, however, 25 g/d, or a value comparable to the tensile strength of high strength polyethylene fibers and aramid fibers, would be the upper limit.

Using PVA fibers having a tensile strength of 12 g/d and above, even surgical sutures having a high tensile strength that exceeds 8 g/d can be obtained.

PVA fibers must also have an elongation at break not higher than 6%. If their elongation at break exceeds 6%, a slippage will occur in the fixed area of the periosteum, bone, etc. Preferably, the PVA fibers have an elongation at break not higher than 5.5%. The elongation at break of fibers will usually decrease as they are drawn at higher draw ratios in their production. However, in the case of PVA fibers, about 3.5% residual elongation at break develops even if the draw ratio is increased up to the fiber breaking point, so the lower limit of the elongation at break of the PVA fibers is about 3.5%.

Another requirement for the PVA fibers is that they have a knot strength of at least 4 g/d. As a ligature, both ends of a surgical suture are always tied off and the stress on the suture will be concentrated at the knot. If the knot strength of the PVA fibers is lower than 4 g/d, it is difficult to fix the ligated portion in a satisfactory way. For this reason, the PVA fibers desirably have the highest possible knot strength, preferably at least 5 g/d. The knot strength of a fiber is related to various physical properties such as tensile strength, tensile modulus, fineness and tensile elongation at break, and is not simply proportional to tensile strength. However, in the case of the PVA fibers used in the present invention which have a tensile strength of at least 12 g/d and a tensile elongation at break of no more than 6%, the upper limit of their knot strength is about 8 g/d.

Braids can be made of the above described PVA fibers by any known methods. For instance, they can be made into a braid that is composed of sheaths counting eight or multiples of eight bundles of fibers and cores which number about one half as many as the sheaths.

What is most important in the present invention is that the suture formed into a braid have a knot retention of at least 3 g/d. As already mentioned, the stress on a tied off suture is concentrated at the knot, so if the knot retention is smaller than 3 g/d, slippage will occur to cause the knot to untie. The surgical suture of the present invention is composed of PVA fibers having high friction between fibers and hence has high knot retention. The suture formed into a braid preferably has the highest possible knot retention and in the present invention its value can be increased up to about 8 g/d by a suitable method such as by optimizing the physical properties of the PVA fiber from which the suture is made or by optimizing the braid making process.

For example, the tensile strength of PVA fibers can be increased by drawing them at a slow drawing rate and the use of a silicone based surfactant will prevent fibers from being damaged in the drawing stage.

PVA fibers generally have superior creep characteristics and they exhibit only about 6% creep even if they are subjected to about one tenth of their breaking load for a period of 12 weeks.

The PVA fibers from which the surgical suture of the present invention is made can be produced under optimal conditions in accordance with the dry-wet spinning method described in JP-A-02-300308 or a gel spinning method. For example, PVA preferably having a degree of polymerization of at least 1,500 but not higher than 7,000 (more preferably at least 3,000 and most preferably at least 4,500) and a degree of saponification, although complete saponification is contemplated, of at least 99% is dissolved in a solvent selected from among saturated aliphatic polyhydric alcohols (e.g. glycerin, ethylene glycol and propylene glycol), dimethyl sulfoxide, dimethylformamide, 1,3-dimethyl-2-imidazolidinone, water and mixtures thereof, and the resulting spinning solution is subjected to dry-wet spinning. Any other known method such as a wet spinning method described, for example, in British Patent 1314000 may be employed, in which an aqueous solution of PVA containing boric acid or a borate is used as a spinning solution where a mixture of an alkali hydroxide, sodium sulfate, etc., is used as a coagulating bath.

The degree of polymerization (Pa) defined in this application is determined from the intrinsic viscosity ($[\eta]$) measured according to the method of JIS K-6726 using 0.5 g/dℓ aqueous solution of PVA at a temperature of 30°C. The calculation of the degree of polymerization is as follows:

$$\log (Pa) = 1.613 \times \log([\eta] \times 10^4 / 8.19).$$

The surgical suture of the present invention has an extremely high tensile strength and a low creep characteristic and yet it is excellent in knot retention. This is because it is composed of flexible PVA fibers that can be prepared at a fairly low cost, which fibers have good biocompatibility, and which fibers have an extremely high tensile strength and a low creep characteristic, properties that are absent from polyesters and polypropylene as are conventionally used as general purpose synthetic fiber materials. In addition, the PVA fibers show such high friction between fibers that only one or two throws of ligation are sufficient to tie off the ends of a suture without slippage. Hence, the surgical suture of the present invention exhibits outstandingly high knot retention.

The tensile strength, knot strength and elongation at break of the surgical suture of the present invention are measured in accordance with the methods described in JIS L-1013 using gauge length of 25 cm and pulling speed of 30 cm/min. Knot retention measurement is conducted by the following method: two sutures each in the form of a braid about 30 cm long were swollen in physiological saline and their opposing ends were fixedly knotted by one throw; the sutures were fixed between an upper and a lower chuck that were 25 cm apart, with the knot being positioned equidistantly from the two chucks; the sutures thus tied off were stretched at a speed of 30 cm/min to yield a load-elongation curve; the peak load on this curve was designated as the knot retention.

For creep value measurement, a sample 30 cm long was used and its length ($L_0$) is first measured under a load that is about one tenth of the breaking load. Then, the sample is left to stand in a preconditioned atmosphere (20°C x 65% R.H.) for 12 weeks, and its length ($L_1$) is again measured. The creep value is defined by the following equation:

$$\text{Creep value (\%)} = (L_1 - L_0)/L_0 \times 100.$$

## Example 1

A spinning solution formed of PVA (degree of polymerization: 4,000; degree of saponification: 99.9 mol%) dissolved in dimethyl sulfoxide at a concentration of 14.5 wt% based on total solution weight was subjected to dry-wet spinning in a coagulating bath consisting of methanol (87 part) and dimethyl sulfoxide (13 part), having a temperature of 18°C. The spun filaments were thermally drawn at a temperature of 250°C at a draw ratio of 15.5 x to prepare a bundle of PVA filaments having a fineness of 100 d per 10 f. The fiber bundle had a tensile strength of 16 g/d, an elongation at break of 4.9% and a knot strength of 5.3 g/d and a creep value of 0.5%. Cores consisting of four of such fiber bundles (400 d per 40 f) and sheaths consisting of eight of them were braided to make a suture having a diameter of about 0.6 mm. The suture had a knot retention of 4.2 g/d and was entirely free from knot slippage.

The suture was implanted under the dorsal skin of laboratory rats for 9 weeks and thereafter recovered. Its strength was as high as 80% of the initial value, demonstrating its excellent retention performance.

## Comparative Example 1

A commercial grade of high strength polyethylene fibers were braided as in Example 1 and the knot retention of the resulting suture was measured. Under a stress of only 1.2 g/d, the knot began to slip and eventually untied.

## Examples 2 and 3

PVA having a degree of polymerization of 1,700 (Example 2) or 5,100 (Example 3), each having the degree of saponification of 99.9 mol%, was dissolved in dimethyl sulfoxide at a concentration of 24 wt% (Example 2) or 12 wt% (Example 3) to yield a spinning solution.

Each of the thus prepared spinning solutions was subjected to dry-wet spinning in a methanol containing coagulating bath by passage through a 25-mm air gap from a spinneret consisting of 40 fine stainless steel cylinders (i.d.: 0.5 mm) that were embedded to protrude by 3 mm at the exit end of the spinning solution.

After dimethyl sulfoxide was removed with methanol, the filaments were dried and taken up to obtain undrawn yarns. The yarns were then drawn in a hot air heating bath 3 m long that was set to have an entrance temperature of 205°C and an exit temperature of 255°C.

From the PVA having a degree of polymerization of 1,700, a bundle of PVA fibers having a fineness of 130 d per 40 f was obtained by drawing at a draw ratio of 18.3; the bundle had a tensile strength of 12.8 g/d, an elongation at break of 5.2% and a knot strength of 4.1 g/d.

From the PVA having a degree of polymerization of 5,100, a bundle of PVA fibers having a fineness of 80 d per 40 f was obtained by drawing at a draw ratio of 15.3; the bundle had a tensile strength of 17.8 g/d, an elongation at break of 4.9% and a knot strength of 5.8 g/d.

Eight of the PVA fiber bundles having a fineness of 130 d per 40 f were used as sheaths and braided to make a suture without cores. On the other hand, cores consisting of four of the PVA fiber bundles having a fineness of 80 d per 40 f and sheaths consisting of eight of the same fiber bundles (finenes: 80 d per 40 f) were braided to make another suture. The knot retention of the two sutures was 3.2 g/d and 4.9 g/d, respectively, and knot slippage was entirely absent from these sutures.

## Comparative Example 2

The procedure of Example 2 was repeated using a spinning solution that had PVA (degree of polymerization: 1,300) dissolved in dimethyl sulfoxide at a concentration of 26 wt%. The resulting bundle of PVA fibers had a tensile strength of 11.0 g/d, an elongation at break of 5.1% and a knot strength of 3.5 g/d. The knot retention of a suture made from said fiber bundle was only 2.7 g/d.

## Examples 4 and 5

PVA having a degree of polymerization of 3,300 (Example 4) or 5,100 (Example 5) was mixed with 2.1% boric acid (aqueous) and thereafter adjusted to a pH of 4.2 to make a spinning solution.

The resulting two spinning solutions were wet spun by being extruded through two spinnerets, one having 40 holes (Example 4) and the other having 100 holes (Example 5), at a spinning draft of 0.25 into a coagulating bath containing sodium sulfate and sodium hydroxide at respective concentrations of 350 g/ℓ and 40 g/ℓ. Thereafter, the spun filaments were neutralized by immersion in a neutralizing bath consisting of sodium sulfate (250 g/ℓ) and sulfuric acid (30 g/ℓ) while they were drawn at a draw ratio of 4.5. The drawn yarns were washed with water and dried. The dried yarns were further drawn at a draw ratio of 4 in a hot air heating furnace 5 m long that was set to have an internal temperature of 245°C.

As a result, two bundles of PVA fibers were obtained; one of them had a fineness of 80 d per 40 f, a tensile strength of 13.5 g/d, an elongation at break of 5.0% and a knot strength of 5.4 g/d, whereas the other fiber bundle had a fineness of 100 d per 100 f, a tensile strength of 15.6 g/d, an elongation at break of 5.2%, and a knot strength of 5.9 g/d.

Sutures were made from these bundles of PVA fibers as in Example 1. Their performance was excellent as evidenced by knot retentions of 4.1 g/d and 4.5 g/d.

The surgical suture of the present invention has an extremely high tensile strength and a low creep characteristic which are not possessed by conventional polyester and polypropylene materials. It is also flexible and has good biocompatibility. In addition, its knot retention is so high that only one or two throws of ligation are sufficient to tie off both ends of the suture without any potential knot slippage.

## Claims

1. A polyvinyl alcohol surgical suture that comprises a braid of polyvinyl alcohol fibers having a tensile strength of from 12 to 25 g/d, a tensile elongation at break of from 3.5 to 6% and a knot strength of from 4 to 8 g/d, said suture having a knot retention of from 3 to 8 g/d.

2. A surgical suture according to claim 1, wherein said polyvinyl alcohol fibers have a tensile strength of at least 14 g/d.

3. A surgical suture according to claim 1, wherein said polyvinyl alcohol fibers have a tensile strength of at least 16 g/d.

4. A surgical suture according to claim 1, wherein said polyvinyl alcohol fibers have a knot strength of at least 5 g/d.

5. A surgical suture according to claim 1, which has a knot retention of at least 4 g/d.

6. A surgical suture according to claim 1, wherein said polyvinyl alcohol fibers have a creep value of no more than 10%.

7. A surgical suture according to claim 1, wherein said polyvinyl alcohol fibers comprise a polyvinyl alcohol having a degree of polymerization of from 1,500 to 7,000.

8. A surgical suture according to claim 7, wherein said polyvinyl alcohol fibers comprise a polyvinyl alcohol having a degree of polymerization of at least 3,000.

9. A surgical suture according to claim 8, wherein said polyvinyl alcohol fibers comprise a polyvinyl alcohol having a degree of polymerization of at least 4,500.

## Patentansprüche

1. Chirurgisches Nahtmaterial aus Polyvinylalkohol, das eine Flechte aus Polyvinylalkohol-Fasern umfaßt, welche eine Zugfestigkeit von 12 bis 25 g/d, eine Bruchdehnung von 3,5 bis 6 % und eine Knotenfestigkeit von 4 bis 8 g/d haben; wobei das Nahtmaterial eine Knotenretention von 3 bis 8 g/d hat.

2. Chirurgisches Nahtmaterial nach Anspruch 1, in dem die Polyvinylalkohol-Fasern eine Zugfestigkeit von mindestens 14 g/d haben.

3. Chirurgisches Nahtmaterial nach Anspruch 1, in dem die Polyvinylalkohol-Fasern eine Zugfestigkeit von mindestens 16 g/d haben.

4. Chirurgisches Nahtmaterial nach Anspruch 1, in dem die Polyvinylalkohol-Fasern eine Knotenfestigkeit von mindestens 5 g/d haben.

5. Chirurgisches Nahtmaterial nach Anspruch 1, welches eine Knotenretention von mindestens 4 g/d hat.

6. Chirurgisches Nahtmaterial nach Anspruch 1, in dem die Polyvinylalkohol-Fasern einen Kriechwert von nicht mehr als 10 % haben.

7. Chirurgisches Nahtmaterial nach Anspruch 1, in dem die Polyvinylalkohol-Fasern einen Polyvinylalkohol mit einem Polymerisationsgrad von 1500 bis 7000 erhalten.

8. Chirurgisches Nahtmaterial nach Anspruch 7, in dem die Polyvinylalkohol-Fasern einen Polymerisationsgrad von mindestens 3000 haben.

**9.** Chirurgisches Nahtmaterial nach Anspruch 8, in dem die Polyvinylalkohol-Fasern einen Polyvinylalkohol mit einem Polymerisationsgrad von mindestens 4500 erhalten.

## Revendications

**1.** Fil de suture chirurgicale en poly(alcool vinylique), qui comprend une tresse de fibres de poly(alcool vinylique) ayant une résistance à la traction comprise entre 12 et 25 g/d, un allongement en traction à la rupture compris entre 3,5 et 6 % et une résistance de noeud comprise entre 4 et 8 g/d, ledit fil de suture ayant un pouvoir de conservation de noeud compris entre 3 et 8 g/d.

**2.** Fil de suture chirurgicale selon la revendication 1, dans lequel lesdites fibres de poly(alcool vinylique) ont une résistance à la traction d'au moins 14 g/d.

**3.** Fil de suture chirurgicale selon la revendication 1, dans lequel lesdites fibres de poly(alcool vinylique) ont une résistance à la traction d'au moins 16 g/d.

**4.** Fil de suture chirurgicale selon la revendication 1, dans lequel lesdites fibres de poly(alcool vinylique) ont une résistance de noeud d'au moins 5 g/d.

**5.** Fil de suture chirurgicale selon la revendication 1, lequel présente un pouvoir de conservation de noeud d'au moins 4 g/d.

**6.** Fil de suture chirurgicale selon la revendication 1, dans lequel lesdites fibres de poly(alcool vinylique) ont un degré de fluage inférieur ou égal à 10 %.

**7.** Fil de suture chirurgicale selon la revendication 1, dans lequel lesdites fibres de poly(alcool vinylique) comprennent un poly(alcool vinylique) ayant un degré de polymérisation compris entre 1 500 et 7 000.

**8.** Fil de suture chirurgicale selon la revendication 7, dans lequel lesdites fibres de poly(alcool vinylique) comprennent un poly(alcool vinylique) ayant un degré de polymérisation d'au moins 3 000.

**9.** Fil de suture chirurgicale selon la revendication 8, dans lequel lesdites fibres de poly(alcool vinylique) comprennent un poly(alcool vinylique) ayant un degré de polymérisation d'au moins 4 500.

FIG. 1

FIG. 2